# EUROPEAN PATENT SPECIFICATION

(11) **EP 1 945 226 B1**
(45) Date of publication and mention of the grant of the patent: **21.09.2016**
(21) Application number: 06747728.1
(22) Date of filing: 27.03.2006
(51) Int. Cl.: A61K 31/616, A61K 31/167, A61K 31/4402, A61K 31/522, A61K 9/20, A61K 9/32, A61P 25/06, A61P 29/00, A61P 11/02

(54) **DRUG COMBINATION WITH ANALGESIC, ANTI-INFLAMMATORY AND DECONGESTIVE ACTIVITY**
ARZNEIMITTELKOMBINATION MIT ANALGETISCHER, ENTZÜNDUNGSHEMMENDER UND DEKONGESTIVER WIRKUNG
ASSOCIATION MEDICAMENTEUSE A ACTIVITE ANALGESIQUE, ANTI-INFLAMMATOIRE ET DECONGESTIONNANTE

(30) Priority: 20.04.2005 RO 200500371
(43) Date of publication of application: 23.07.2008
(73) Proprietor: Voicu, A. Victor, 011256 Bucuresti (RO); Jiquidi, Marilena, 011256 Bucuresti (RO); Mircioiu, Constantin, 050528 Bucuresti (RO)
(72) Inventor: Voicu, A. Victor, 011256 Bucuresti (RO); Jiquidi, Marilena, 011256 Bucuresti (RO); Mircioiu, Constantin, 050528 Bucuresti (RO)
(74) Representative: Popa, Cristina
(86) International application number: PCT/RO2006/000007
(87) International publication number: WO 2006/112744

(56) References cited:
- RO-B1- 102 954
- BOONKERD S ET AL: "Separation and simultaneous determination of the components in an analgesic tablet formulation by micellar electrokinetic chromatography" JOURNAL OF CHROMATOGRAPHY A, ELSEVIER, AMSTERDAM, NL, vol. 695, no. 1, 24 March 1995 (1995-03-24), pages 97-102, XP004022937 ISSN: 0021-9673
- DATABASE WPI 14 November 1995 (1995-11-14), Derwent Publications Ltd., London, GB; Class 960,page 3, AN 1996-026969 XP002409257 YAMASHITA E: "Anti-inflammatory drug - contains astaxanthin di:ester(s) extracted from krill" -& JP 07 300421 A (ITANO REITO KK) 14 November 1995 (1995-11-14)
- MANSFIELD L E: "THE ROLE OF ANTIHISTAMINE THERAPY IN VASCULAR HEADACHES" JOURNAL OF ALLERGY AND CLINICAL IMMUNOLOGY, MOSBY - YEARLY BOOK, INC, US, vol. 86, October 1990 (1990-10), pages 673-676, XP000979021 ISSN: 0091-6749
- LIPTON R B ET AL: "Efficacy and safety of acetaminophen, aspirin, and caffeine in alleviating migraine headache pain: three double-blind, randomized, placebo-controlled trials." ARCHIVES OF NEUROLOGY. FEB 1998, vol. 55, no. 2, February 1998 (1998-02), pages 210-217, XP008071828 ISSN: 0003-9942
- "Vidal 1997: RUMICINE" 1997, EDITIONS DU VIDAL , PARIS 73 , XP002408659 page 1460, column 1, paragraph 1-3
- "Rote Liste 2003: Melabon No. 05 434" 2003, ROTE LISTE SERVICE GMBH , FRANKFURT/MAIN , XP002408660
- SOMERVILLE B W: "Treatment of migraine attacks with an analgesic combination (Mersyndol)." THE MEDICAL JOURNAL OF AUSTRALIA. 5 JUN 1976, vol. 1, no. 23, 5 June 1976 (1976-06-05), pages 865-866, XP008071865 ISSN: 0025-729X

## Description

The invention concerns a drug combination with analgesic, anti-Inflammatory and decongestant action, indicated in therapy of various somatic pains related to lumbar discopathy and/or lumbago.

Currently, various analgesic - anti-inflammatory formulations are known; they associate acetylsalicylic acid with pyrazolonic derivatives, decongestants such as phenylephrine, antihistaminic compounds, caffeine, etc.

A further drug combination with similar action, also discovered by the authors, associates acetylsalicylic acid with paracetamol, pheniramine and caffeine and is formulated as tablets (Patent RO no. 102954). Although the therapeutic results obtained with this combination are good, the stability studies show a physico-chemical interaction between pheniramine and the other compounds, that accelerates the decomposition of aspirin and paracetamol.

Another patent of the authors (RO no. 106333) associates acetylsalicylic acid with paracetamol, chlorpheniramine (antihistaminic compound) and theophylline. The combination is more stable than the previous, but some of the therapeutic effects are weaker, at the same doses, comparing with the combination that includes caffeine.

A Japanese publication by Yamashita "Anti-inflammation drug contains astaxanthin di:ester(s) extracted from krill" describes an anti-inflammatory combination for common cold, comprising 0.5% chlorpheniramine maleate, 20.5 % acetaminophen,6.0% anhydrous caffeine, 20.0% acetylsalicylic acid, 3.0% carbetabentane citrate, 14% guaiacol potassium sulphate, 9.0% hydrocellulose, 1.0% magnesium stearate and 26% astaxanthin diester.

Our invention proposes a drug combination characterized in that it comprises a combination of:
- 100-250 mg acetylsalicylic acid,
- 75-150 mg para-acetylaminophenol,
- 1-5 mg chlorpheniramine,
- 20-50 mg caffeine, and pharmaceutically acceptable excipients,
for use In the treatment of pain related to inflammatory processes wherein pain is related to lumbar discopathy and/or lumbago.

This drug combination is a synergic combination of 100-125 mg acetylsalicylic acid, 75-200 mg para-acetylaminophenol, 1-5 mg chlorpheniramine and 20-50 mg caffeine (base or salt), formulated as tablets or gastric-enterosoluble dragees with usual excipients.

This invention associates an analgesic (acetylsalicylic acid) with another analgesic substance (paracetamol), plus an antihistaminic compound (chlorpheniramine) and caffeine (that triggers the increase of the vascular tonus in cerebral circulation; counters the sedative effect of chlorpheniramine; improves the oral bioavailability of the associated compounds).

The result is a combination with a strong potency of the anti-inflammatory effect (as experimentally proved), as well as decongestant and analgesic effects.

The drug combination has a strong and prolonged analgesic and anti-inflammatory action, but also a great stability.

In the following, the anti-inflammatory and therapeutic effects for the combination will be shown.

### The anti-Inflammatory effects of our drug combination in rats

### Method:

The anti-inflammatory effect was found via the experimental inflammation model with carrageenan at the rats' posterior paws. For the experimental research, we used Wistar rats with 120±10g weight, in groups of 9 animals.

1% carrageenan saline solution was administered by subcutaneous injection (0.1 mL volume) In the posterior paw.

The anti-inflammatory combination was administered 1 hour before carrageenan. The posterior paws' dimensions were measured before and 3 hours after carrageenan solution injection. The contra lateral paw was used as reference. The following combination was used for the experiments:

| | |
|---|---|
| Acetylsalicylic acid | 1.25 g |
| Paracetamol | 0.75 g |
| Chlorpheniramine | 0.03 g |
| Natriumbenzoic Caffeine | 0.15 g |

This (combination, formulated as) suspension was administered orally (gavage), in dosage of 0.2 mL/100 g rat body weight.

Acetylsalicylic acid was administered independently, in doses of 250 mg/kg body weight, equivalent to the quantity used in the combination. Chlorpheniramine was also (independently) administered by oral route, as reference, in doses equivalent to 6 mg/kg body weight.

### Results:

1. the anti-inflammatory effect of the combination was characterized by the disappearance of edema induced by carrageenan (average = 92-112%)
2. acetylsalicylic acid produces an anti-inflammatory effect (reduces pow edema) with mean value of approximately 22%
3. chlorpheniramine has no anti-inflammatory effect in the dosage used

Normal control was the first reference = 100%
Control with carrageenan= 177%,
Dispersion = 168% to 196%

### Evaluation of therapy with our drug combination in patients with medium and weak algesic syndrome

**Objective:** The clinical study objective was clinical testing of the analgesic effects produced by our combination, with tablets as pharmaceutical formulation. The following combination was used: acetylsalicylic acid, paracetamol, chlorpheniramine maleate and caffeine (125:75:3:15).

**Method:** The clinical experiment was open, conducted in ambulatory, on 12 patients in need of a short time treatment for light or medium pains with various pathogenesis.

The main indications for the analgesic treatment were:
- migraine syndrome,*
- radicular syndrome,
- direct neuralgia,
- vascular cephalalgia,*
- lumbar discopathy,
- lumbago,
- viral cephalalgia.*

### (* reference example)

The product was administered in dosage of 2 tablets/day. The effect was quantified by patients on a 0-5 scale (meaning: 0=no effects, 5= very good effects).

**Results:** The results are shown in Table 1 below.

Good and very good results were obtained at 58.3% of patients (7 individuals), while in 8.3% (1 case) the result was quantified as medium. No response was obtained in 25% of patients (3 persons). The results of the study proved also that the combination is well tolerated.

Major adverse reactions appeared only in one case. Nausea and vomitus appeared at one patient with previous cicatricial duodenum ulcer. Also, in one case we had a very good response but dizziness and unsteadiness for about 1 hour.

**Table 1: Results of therapy In patients with medium and weak algesic syndrome**

| **No.** | **Name** | **Age (years)** | **Diagnostic** | **Result** |
|---|---|---|---|---|
| 1 | P.F. | 55 | common migraine | 5 |
| 2 | S.M. | 56 | radicular syndrome T4 | The patient accuses nausea and vornitus after 2 tablets. In his history, the patient had inactive chronic cicatricial duodenum ulcer. |
| 3 | T.I. | 20 | neuralgia of steno-palatal ganglia | 0 |
| 4 | G.S: | 49 | vascular cephalalgia associated with H T A | 4 |
| 5 | A.F. | 51 | common migraine | 5 |
| 6 | AR. | 21 | common migraine | 5 |
| 7 | P.G. | 20 | common migraine | 4 |
| 8 | D.I. | 20 | trigeminal neuralgia | 0 |
| 9 | G.I. | 20 | lumbar discopathy (with clinical and radiological changes) | 3 |
| 10 | S.D. | 20 | lumbago (without clinical and radiological changes) | 4 |
| | | | | 5 |
| 11 | B.E. | 36 | cephalalgia associated with pulmonary virosis | dizziness and unsteadiness for about 1 hour |
| 12 | B.F. | 33 | common migraine | 0 |

### Pharmaceutical formulations of our drug combination

**Example1.** In order to obtain 1000 tablets of 250 mg, 100 g acetylsalicylic acid (aspirin) and 15 g starch are granulated with 50 mL agglutinant solution; separately, 75 g paracetamol (para-acetylaminophenole) is granulated together with 20g caffeine and 12 g starch using 1O mL agglutinant solution. A third mixture is granulated as following: 1 g chlorpheniramine and 1.8 g potassium meta-bisulfite, with 5 mL agglutinant solution.

As agglutinant, we use a solution 6% (w/v) polyvinylpirolidone water-alcohol solution (Kollidon 30) or a gelatine solution.

The three granulated mixtures are dried at 40°C, for 24 hours, then homogenized with 4 g magnesium stearate and compressed in order to obtain tablets with 250 mg weight.

**Example 2.** In order to obtain 1000 tablets of 500 mg, we used 500 g acetyl-salicylic acid, 150 g paracetamol, 50 g caffeine, 5 g chlorpheniramine, other components and techniques as described in example 1 above.

The tablets can be coated by spraying with the following suspension: 5 % cellulose acetophthalate, 1 % polyethylenglycol 4000, 3.5 % talc, 1.5 % titanium dioxide and 0.3 % triton X -100 (or Tween 80) in dioxane-acetone mixture as solvent. For a non-enterosoluble coat, cellulose aceto phthalate must be replaced with hydroxy-methyl-propyl-cellulose.

The product can be prepared also in slow release formulations by inclusion in a polyethylene inert matrix.

The analgesic, anti-inflammatory and decongestant effects were tested in order to compare the combination of the components versus each component separately: acetylsalicylic acid, paracetamol and chlorpheniramine. The anti-inflammatory effect was experimentally proved on the inflammation with carrageenan in rats.

Also, in preliminary clinical experiments, the intense analgesic and anti-inflammatory efficiency was proved in cephalalgia with various origins, migraine syndrome, different somatic pains, rhino-sinusal congestions, etc.

## Claims

1. A drug combination **characterized in that** it comprises a combination of:
- 100 - 250 mg acetylsalicylic acid,
- 75 - 150 mg para-acetylaminophenol,
- 1 - 5 mg chlorpheniramine,
- 20 - 50 mg caffeine, and pharmaceutically acceptable excipients,
for use in the treatment of pain related to inflammatory processes wherein pain is related to lumbar discopathy and/or lumbago.

2. Combination for use according to claim 1, **characterized in that** said drug combination comprises:
- 125 mg acetylsalicylic acid (aspirin),
- 75 mg para-acetylaminophenol (paracetamol),
- 15 mg anhydrous caffeine,
- 2 mg chlorpheniramine maleate.

## Patentansprüche

1. Pharmazeutische Zusammensetzung, **dadurch gekennzeichnet, daß** diese eine Kombination von:
- 100-250 mg Acetylsalicylsäure,
- 75-150 mg para-Acetylaminophenol,
- 1-5 mg Chlorpheniramin,
- 20 bis 50 mg Koffein
und pharmazeutisch annehmbaren Hilfsstoffen enthält und für die Behandlung von Schmerzen und entzündlichen Prozessen zur Verwendung kommt wobei die Schmerzen mit Lendenwirbelsäule-Diskopathie und / oder Lumbago verbunden sind.

2. Pharmazeutische Zusammensetzung zur Anwendung gemäß Anspruch 1, **dadurch gekennzeichnet, daß** diese pharmazeutische Zusammensetzung:
- 125 mg Acetylsalicylsäure (Aspirin),
- 75 mg para-Acetylaminophenol (Paracetamol),
- 15 mg Koffein,
- 2 mg Chlorpheniramin-maleat, enthält.

## Revendications

1. Une combinaison de substances-actives comprenant une combinaison de:
- 100 à 250 mg d'acide acétylsalicylique,
- 75 à 150 mg de para-acetylaminophenole,
- 1 à 5 mg de chlorphénamine,
- 20 à 50 mg de caféine et des excipients pharmaceutiquement acceptables utilisée dans le traitement de la douleur issue des processus inflammatoires engendrant des douleurs de type discopathie et/ou lumbago

2. Combinaison à être utilisée selon la revendication 1, **caractérisée par** ce qu'elle comprend:
- 125 mg d'acide acétylsalicylique (aspirine),
- 75 mg de para-acetylaminophenole (paracétamol),
- 2 mg de chlorphénamine maleate,
- 15 mg de caffeine.
